## Europäisches Patentamt
## European Patent Office
## Office européen des brevets

(19)

(11) Publication number: **0 000 218**
**B1**

(12)

# EUROPEAN PATENT SPECIFICATION

(45) Date of publication of patent specification: **11.11.81**

(51) Int. Cl.³: **C 07 D 323/00**

(21) Application number: **78200037.6**

(22) Date of filing: **12.06.78**

(54) Process for the isolation of 1,4,7,10,13,16-hexaoxacyclooctadecane.

(30) Priority: **23.06.77 GB 2633377**

(43) Date of publication of application:
**10.01.79 Bulletin 79/1**

(45) Publication of the grant of the European patent:
**11.11.81 Bulletin 81/45**

(84) Designated Contracting States:
**CH DE FR GB NL SE**

(56) References cited:
**CHEMICAL ABSTRACTS, vol. 86 (1977) 88.648b,
page 446.
Columbus Ohio**

(73) Proprietor: **SHELL INTERNATIONALE RESEARCH
MAATSCHAPPIJ B.V.
Carel van Bylandtlaan 30
NL-2596 HR DEN HAAG (NL)**

(72) Inventor: **De Jong, Feike
Badhuisweg 3
NL-1031 CM Amsterdam (NL)**
Inventor: **Reinhoudt, David Nicolaas
Badhuisweg 3
NL-1031 CM Amsterdam (NL)**

(74) Representative: **Keuzenkamp, Abraham et al,
P.O. Box 302
NL-2501 CH Den Haag (NL)**

Courier Press, Leamington Spa, England.

**0 000 218**

## Process for the isolation of 1,4,7,10,13,16-hexaoxacyclooctadecane

The invention relates to a process for the isolation of 1,4,7,10,13,16-hexaoxacyclooctadecane from a mixture also containing one or more other macrocyclic polyethers.

Hereinafter 1,4,7,10,13,16-hexaoxacyclooctadecane will also be referred to by its trivial name "18-crown-6". Its structural formula is shown on the Formula page (compound 9).

18-Crown-6 can be prepared by heating tetraethylene glycol and bis(2-chloroethyl) ether in the presence of tetrahydrofuran as a solvent and potassium hydroxide, see "Synthesis" 1976, 515—516. The reaction mixture thus formed contains 18-crown-6, potassium chloride, water and by-products, i.a. macrocyclic polyethers having a ring of larger size than 18-crown-6. The solvent was evaporated from the reaction mixture to give a brown slurry to which dichloromethane was added. The potassium chloride was filtered off from the solution thus obtained, the filtrate was dried ($MgSO_4$), the solvent was evaporated from the dried filtrate to yield a residue of crude 18-crown-6. This residue was distilled to give a discoloured distillate containing 18-crown-6. This distillate was dissolved in acetonitrile and the solution was cooled to −45°C. The resultant precipitate of 18-crown-6-acetonitrile complex was collected by filtration. Distillation of this complex gave a distillate of pure 18-crown-6.

A disadvantage of this procedure is that the 18-crown-6 is distilled twice and that expensive measures must be taken to prevent the occurrence of powerful and destructive explosions during these distillations, see "Chemical & Engineering News", September 6, 1976, page 5 and December 13, 1976, page 5. Moreover the 18-crown-6 is obtained in a fairly low yield. It is also known, e.g. from Z. Naturforsch 31b 1192—1200 (1976) that 18-crown-6 forms a crystalline adduct with nitromethane.

The Applicants have found a process for the isolation of 18-crown-6- in which the 18-crown-6 is obtained in high yield and need not be distilled, thus avoiding the explosions mentioned hereinbefore.

Accordingly the invention provides a process for the isolation of 1,4,7,10,13,16-hexaoxacyclooctadecane from a mixture also containing one or more other macrocyclic polyethers containing rings of more than 7 carbon atoms, characterized by reacting the 1,4,7,10,13,16-hexaoxacyclooctadecane with nitromethane in the presence of a solvent for the said macrocyclic polyethers, to precipitate a complex of 1,4,7,10,13,16-hexaoxacyclooctadecane and nitromethane, separating precipitated complex from the mixture containing the complex, dissociating separated complex and separating the components from each other.

Surprisingly, nitromethane is selective in that, when contacted with a mixture containing 18-crown-6 and one or more other macrocyclic polyethers, it only forms a complex with 18-crown-6. Hence, the 18-crown-6-nitromethane complex obtained according to the invention does not, or hardly contains any macrocyclic polyethers other than 18-crown-6.

As the 18-crown-6-nitromethane complex gives off nitromethane vapour the complex can simply be dissociated into 18-crown-6 and nitromethane by allowing it to stand in the open air. The complex is rapidly dissociated by heating it at sub-atmospheric pressure and removing the nitromethane vapour formed, leaving pure or almost pure 18-crown-6. Heating may take place at a temperature of, for example, 35°C to 125°C at a pressure of, for example, 5 to 1500 Pa.

The process according to the invention must be carried out in a solvent for macrocyclic polyethers in which the 18-crown-6-nitromethane complex can be precipitated. Such a solvent may substantially consist of nitromethane. A solvent is taken to consist substantially of a specified compound when the content of this specified compound in the solvent is higher than 50%w.

According to a feature of the invention the solvent substantially consists of an ether (other than a macrocyclic polyester having more than 7 carbon atoms in the ring). Preferred ethers are dialkyl ethers and cyclic ethers. Examples of dialkyl ethers are diethyl ether, di-n-butyl ether and di-n-hexylether. The 18-crown-6-nitromethane complex is considerably less soluble in diethyl ether than in nitromethane at the same temperature, so that a considerably larger part of the 18-crown-6 present in the starting mixture can be isolated. Attractive examples of the cyclic ethers are 1,4-dioxane and tetrahydrofuran.

According to another feature of the invention the solvent substantially consists of an alkanol, a hydrocarbon (or a mixture of hydrocarbons) or water. Examples of suitable alkanols are those having in the range of from one to five carbon atoms, for example methanol, ethanol, 2-propanol and tert-butanol. Examples of suitable hydrocarbons are benzene, toluene, the three xylenes and heptane. Particularly attractive solvents are methanol and benzene. 18-Crown-6, dissolved in water may be removed therefrom by reaction with nitromethane to form a precipitate of the 18-crown-6-nitromethane complex in nitromethane-containing water. Mixtures of solvents may be used, for example of diethyl ether and ethanol.

The 18-crown-6-acetonitrile complex is very difficult to precipitate from the solvents mentioned in the last three paragraphs, if at all, as this complex is too highly soluble in these solvents.

The reaction between 18-crown-6 and nitromethane is preferably carried out at a temperature in the range of from −25°C to +35°C, and, when the solvent substantially consists of nitromethane, of from 0°C to −25°C, so as to enable a very large part of the 18-crown-6 to be isolated. Where the solvent substantially consists of an ether, an alkanol, a hydrocarbon (or a mixture of hydrocarbons) or water, the reaction between 18-crown-6 and nitromethane is very suitably carried out at a tempera-

2

**0 000 218**

ture in the range of from 0°C to +35°C. Ambient Temperature, for example, a temperature between 15°C and 25°, is very suitable.

The 18-crown-6-nitromethane complex can easily be separated from the mixture containing the complex, for example by filtration, centrifugation or decantation.

The 18-crown-6-nitromethane complex contains two molecules of nitromethane per molecule of 18-crown-6. Accordingly, the 18-crown-6 is reacted with nitromethane using a molar ratio of nitromethane to 18-crown-6 of at least 2:1. Preferably, this molar ratio is not higher than 20:1. This range is not critical, molar ratios of nitromethane to 18-crown-6 outside this range may be used, if desired.

The 18-crown-6-nitromethane complex may be precipitated in any suitable manner, for example by adding nitromethane to the starting mixture and, if desired, cooling the mixture thus obtained to a suitable temperature, or by dissolving the starting mixture in diethyl ether and adding nitromethane at ambient temperature to the solution obtained, or by dissolving the starting mixture in any other of the above-mentioned solvents, for example methanol, adding nitromethane and, if desired, cooling the mixture obtained to a suitable temperature. The starting mixture may be solid or liquid at the temperature at which the 18-crown-6 is reacted with nitromethane.

The 18-crown-6 in the starting mixture may have been formed by any process. Very good results have been obtained by reacting tetraethylene glycol with a bis(2-haloethyl)ether, halo representing chloro, bromo or iodo, in the presence of an alkali metal hydroxide. If desired, the alkali metal halide formed and solvent, if present, may be removed from the reaction mixture obtained, leaving the 18-crown-6-containing starting mixture. Other examples of processes for the preparation of 18-crown-6 are:

(1) Elimination of hydrogen chloride from 17-chloro-3,6,9,12,15-pentaoxaheptadecanol, followed by ring closure, in the presence of potassium tert-butoxide, see British patent specification 1,285,367.

(2) Catalytic oligomerization of ethylene oxide, see German Offenlegungsschrift 2,401,126.

(3) Reaction of triethylene glycol with 3,6-dioxa-1,8-dichlorooctane in the presence of potassium hydroxide and 10% aqueous tetrahydrofuran, as described in J.Org.Chem. *39* (1974) 2445—2446.

The invention will now be illustrated by reference to the following Examples.

Preparation of crude 18-crown-6

A 3-litre three-necked round-bottomed flask, fitted with a mechanical stirrer, a reflux condenser and a 250 ml dropping funnel was charged with potassium hydroxide pellets (416 g. containing 6.3 mol KOH), tetraethylene glycol (1.25 mol) and tetrahdyrofuran (1000 ml). The reaction vessel was placed in a heating mantle and gently heated. After 15 minutes a solution of bis(2-chloroethyl)ether (3.125 mol) in tetrahydrofuran (150 ml) was added in one stream from the dropping funnel to the vigorously stirred reactants. The reaction mixture was then heated under reflux, with stirring for 18 hours. Subsequently, the reaction mixture was cooled and the solvent was removed under a pressure of 1.9 kPa to give a brown slurry to which dichloromethane (750 ml) was added. The resulting suspension of potassium chloride was filtered and the potassium chloride filtered off was washed with dichloromethane (100 ml). The combined filtrate and washings were dried over anhydrous magnesium sulphate and the solvent was evaporated at a pressure of 1.9 kPa to give a residue of crude 18-crown-6 (396 g) containing 0.531 mol of 18-crown-6 (yield 42.5%, calculated on starting tetraethylene glycol), potassium chloride and at least eight other compounds among which 1,4,7,10,13,16,19,22,25,28,31,34-dodecaoxacyclohexatriacontane, hexaethyleneglycol and unreacted tetraethylene glycol. This crude 18-crown-6 was used as described hereinafter.

## Example I

*Isolation of 18-crown-6 in nitromethane at —20°C*

Crude 18-crown-6 (3.021 g), prepared as described above, was dissolved in nitromethane (4 ml). The solution formed was cooled to —20°C and the 18-crown-6-nitromethane complex precipitated was filtered off (1.248 g). The complex was kept for 30 minutes at 70°C and a pressure of 13 Pa, leaving 18-crown-6 in a yield of 37% calculated on starting tetraethylene glycol, or 87% calculated on 18-crown-6 in the crude 18-crown-6. The purity of the 18-crown-6 was higher than 99.5%.

## Comparative Experiment A

*Isolation of 18-crown-6 in acetonitrile at —20°C*

Crude 18-crown-6 (3.021 g), prepared as described above, was dissolved in acetonitrile (4 ml). The solution formed was cooled to —20°C and the 18-crown-6-acetonitrile complex was filtered off. The complex was kept for 30 minutes at 70°C and a pressure of 13 Pa, leaving 18-crown-6 in a yield of 27% calculated on starting tetraethylene glycol, or 63% calculated on 18-crown-6 in the crude 18-crown-6. Comparison with Example I shows that complexation with nitromethane allows the 18-crown-6 in a much higher yield.

3

### Comparative Experiment B
*Isolation of 18-crown-6 in acetonitrile at —45°C*

This experiment is according to the process described in "Synthesis" 1976, 515—516.

Crude 18-crown-6 (39.6 g), prepared as described above, was distilled at a pressure of 20 Pa to give a distillate (21.3 g) boiling at 140—210°C. This distillate was mixed with acetonitrile (53 ml) at 20°C and the mixture formed was cooled to —45°C. The resultant 18-crown-6-acetonitrile complex was filtered off and subjected to distillation at a pressure of 2 Pa to give a distillate of 18-crown-6 in a yield of 25% calculated on starting tetraethylene glycol, or 59% calculated on 18-crown-6 in the crude 18-crown-6. Comparison with Example I shows that complexation with nitromethane at —20°C allows the 18-crown-6 in a much higher yield than complexation with acetonitrile at —45°C followed by distillation of 18-crown-6.

### Example II
*Isolation of 18-crown-6 in nitromethane at +20°C*

Crude 18-crown-6 (3.04 g), prepared as described above, was mixed with nitromethane (5 ml) at 20°C. After 16 hours the precipitate formed was filtered off and kept for 30 minutes at 70°C and a pressure of 13 Pa, leaving 18-crown-6 in a yield of 28% calculated on starting tetraethylene glycol, or 66% calculated on 18-crown-6 in the crude 18-crown-6. Comparison of this yield with the higher yield obtained in Example I shows the favourable influence of the temperature of —20°C when nitromethane is used as the solvent.

### Example III
*Isolation of 18-crown-6 in diethyl ether at +20°C*

Crude 18-crown-6 (3.5 g), prepared as described above, was extracted with four 20-ml portions of diethyl ether. The residue of potassium chloride thus obtained was filtered off, the four filtrates were combined, and the solvent was evaporated. Then, a mixture of nitromethane (0.7 ml) and 5.1 ml of diethyl ether was added at 20°C, which caused an immediate precipitation of the 18-crown-6-nitromethane complex. The complex precipitated was filtered off and kept for 30 minutes at 70°C and a pressure of 13 Pa, leaving 18-crown-6 in a yield of 37% calculated on starting tetraethylene glycol, or 87% calculated on 18-crown-6 in the crude 18-crown-6. Comparison of this yield with the lower yield of Example II shows the favourable influence of the diethyl ether.

### Example IV
*Isolation of 18-crown-6 in methanol at +20°C*

Crude 18-crown-6 (3.16 g), prepared as described above, was mixed with nitromethane (0.6 ml) and methanol (4.4 ml) at 20°C. After 16 hours the precipitate formed was filtered off and kept for 30 minutes at 70°C and a pressure of 13 Pa, leaving 18-crown-6 in a yield of 13%, calculated on starting tetraethylene glycol, or 31%, calculated on 18-crown-6 in the crude 18-crown-6. Comparison of this yield with the higher yields obtained in the Examples II and III shows that in this case diethyl ether and nitromethane are more attractive solvents than methanol.

### Example V
*Separation of 18-crown-6 from macrocyclic polyether No. 3 in diethyl ether*

Nitromethane (0.6 ml) was added at 20°C to a solution of 18-crown-6 (1.13 mmol) and macrocyclic polyether No. 3 of the formula page (1.15 mmol) in diethyl ether (6 ml). The precipitate formed was filtered off, washed at 20°C with 1 ml of a diethyl ether/nitromethane (10/1 v/v) mixture and dried with dry air at 20°C. The dried precipitate was the 18-crown-6-nitromethane complex. The complex contained 92% of the starting amount of 18-crown-6 and the molar ratio of 18-crown-6 to macrocyclic polyether No. 3 in the dried precipitate was 97:3. The complex was kept for 30 minutes at 70°C and a pressure of 13 Pa leaving 18-crown-6 containing 3% macrocyclic polyether No. 3.

### Examples VI to XI
*Precipitation of the 18-crown-6-nitromethane complex in various solvents*

Nitromethane (1.48 mmol) was added to a solution of 18-crown-6 (0.19 mmol) and macrocyclic polyether No. 3 of the formula page (0.19 mmol) in a solvent (1 ml) at 25°C. The 18-crown-6-nitromethane complex precipitated was filtered off. The filtered material was kept for 30 minutes at 70°C and a pressure of 13 Pa, which caused dissociation into nitromethane, which was sucked off, and 18-crown-6. Then, the 18-crown-6 was weighed and the yield of it on the starting amount of 18-crown-6 was calculated. Six solvents were tested in this manner. Table I presents the results. The purity of the 18-crown-6 was 99% in each of the Examples.

# 0 000 218

## TABLE I

| Example | Solvent | Yield of 18-crown-6, % | The complex crystallized out | |
|---|---|---|---|---|
| | | | immediately | over a period of 1 to 4 h |
| VI | diethyl ether | 92 | yes | — |
| VII | tetrahydrofuran | 52 | — | yes |
| VIII | 1,4-dioxane | 23 | — | yes |
| IX | methanol | 79 | yes | — |
| X | benzene | 45 | — | yes |
| XI | water | 35 | yes | — |

## Comparative Experiment C

*Precipitation of the 18-crown-6-acetonitrile complex in diethyl ether*

Acetonitrile (1.48 mmol) was added to a solution of 18-crown-6 (0.19 mmol) and macrocyclic polyether No. 3 of the formula page (0.19 mmol) in diethyl ether (1 ml) at 25°C. The 18-crown-6-acetonitrile complex crystallized out immediately and was filtered off. It contained 40% of the starting amount of 18-crown-6. Comparison with Example VI shows that nitromethane had complexed a much larger part of the 18-crown-6 than acetonitrile. The purity of the 18-crown-6 was more than 99%.

## Comparative Experiments D to H

*Attempted precipitation of the 18-crown-6-acetonitrile complex in five solvents*

The experiments of Examples VII to XI were carried out with acetonitrile (1.71 mmol) instead of nitromethane (1.15 mmol): In none of the five experiments was a precipitate formed.

## Comparative Experiments I to P

*Attempted preparation of complexes of nitromethane with macrocyclic polyethers other than 18-crown-6*

These eight comparative experiments were carried out with the macrocyclic polyethers listed in Table II. Their structural formulae are shown on the formula page. Each macrocyclic polyether has been given a number which is mentioned in Table II and on the formula page.

## TABLE II

| Comparative Experiment | Macrocyclic polyether | |
|---|---|---|
| | number | name |
| I | 1 | 2,3,11,12-dibenzo- 1,4,7,10,13,16-hexaoxacyclooctadeca-2,11-diene |
| J | 2 | 2,3-benzo-1,4,7,10,13,16-hexaoxacyclooctadeca-2-ene |
| K | 3 | 1,4,7,10,13-pentaoxacyclopentadecane |
| L | 4 | 2,3-benzo-1,4,7,10,13,17,19-heptaoxacycloheneicosa-2-ene |
| M | 5 | 2,5,8,15,18,21-hexaoxatricyclo[20.4.0.0$^{9,14}$]hexacosane |
| N | 6 | 3,5-benzo-1,7,10,13,16-pentaoxacyclooctadeca-3-ene |
| O | 7 | 3,4-benzo-1,6,9,12,15-pentaoxacycloheptadeca-3-ene |
| P | 8 | 1,10-diaza-4,7,13,16,21,24-hexaoxabicyclo/8,8,8/hexacosane |

5

In Comparative Experiment No. I nitromethane (1.15 mmol) was added to a saturated solution of macrocyclic polyether No. 1 in diethyl ether (1 ml) at 25°C. In Comparative Experiments J to P nitromethane was added to a solution of 100 mg of the macrocyclic polyether in diethyl ether at 25°C. In none of the eight experiments was a precipitate formed.

Comparative Experiment Q

*Attempted preparation of a macrocyclic polyether 1-nitromethane complex*

Nitromethane (1.15 mmol) was added at 25°C to a saturated solution of macrocyclic polyether 1 (Table II and formula page) in methanol (1 ml). No precipitate was formed.

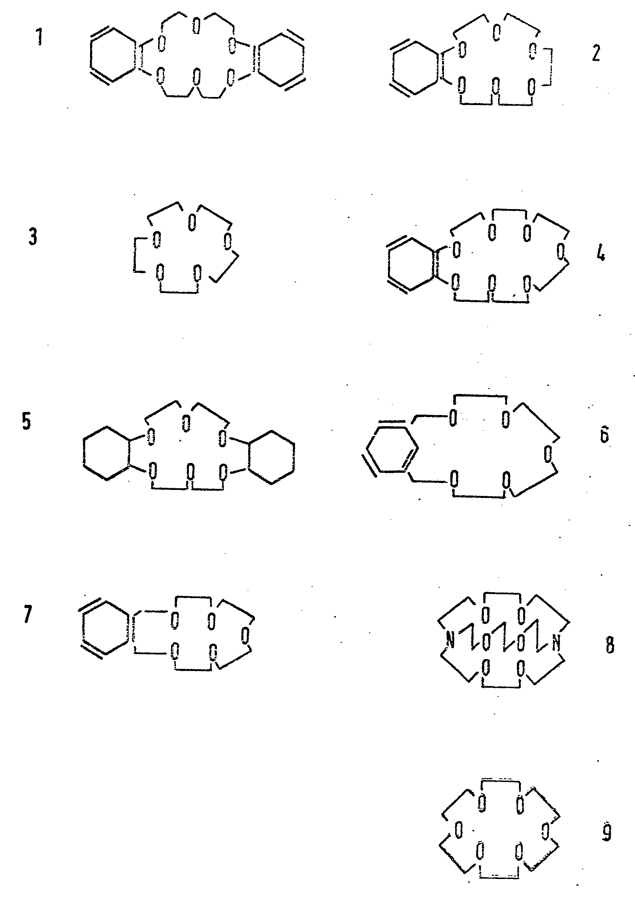

## Claims

1. Process for the isolation of 1,4,7,10,13,16-hexaoxacyclooctadecane from a mixture also containing one or more other macrocyclic polyethers containing rings of more than 7 carbon atoms, characterized by reacting the 1,4,7,10,13,16-hexaoxacyclooctadecane with nitromethane in the presence of a solvent for the said macrocyclic polyethers, to precipitate a complex of 1,4,7,10,13,16-hexaoxacyclooctadecane and nitromethane, separating precipitated complex from the mixture containing the complex, dissociating separated complex and separating the components from each other.

2. Process as claimed in claim 1, characterized in that the solvent comprises greater than 50% by weight of nitromethane.

3. Process as claimed in claim 1, characterized in that the solvent comprises greater than 50% by weight of an ether (other than a macrocyclic polyester having more than 7 carbon atoms in the ring).

4. Process as claimed in claim 3, characterized in that the ether is a dialkyl ether.

5. Process as claimed in claim 4, characterized in that the dialkyl ether is diethyl ether.

6. Process as claimed in claim 3, characterized in that the ether is a cyclic ether.

7. Process as claimed in claim 6, characterized in that the cyclic ether is 1,4-dioxane.

8. Process as claimed in claim 6, characterized in that the cyclic ether is tetrahydrofuran.

9. Process as claimed in claim 1, characterized in that the solvent comprises greater than 50% by weight of an alkanol.

10. Process as claimed in claim 9, characterized in that the alkanol is methanol.

11. Process as claimed in claim 1, characterized in that the solvent comprises greater than 50% by weight of a hydrocarbon or a mixture of hydrocarbons.

12. Process as claimed in claim 11, characterized in that the hydrocarbon is benzene.

13. Process as claimed in claim 1, characterized in that the solvent comprises greater than 50% by weight of water.

14. Process as claimed in any one of the preceding claims characterized in that the reaction between 1,4,7,10,13,16-hexaoxacyclooctadecane and nitromethane is carried out at a temperature in the range of from −25°C to +35°C.

15. Process as claimed in claim 2, characterized in that the reaction between 1,4,7,10,13,16-hexaoxacyclooctadecane and nitromethane is carried out at a temperature in the range of from 0°C to −25°C.

16. Process as claimed in any one of claims 3 to 13, characterized in that the reaction between 1,4,7,10,13,16-hexaoxacyclooctadecane and nitromethane is carried out at a temperature in the range of from 0°C to +35°C.

17. Process as claimed in any one of the preceding claims, characterized in that the 1,4,7,10,13,16-hexaoxacyclooctadecane in the starting mixture has been obtained by reacting tetraethylene glycol with a bis(2-haloethyl)ether in the presence of an alkali metal hydroxide.

## Revendications

1. Procédé pour l'isolement de 1,4,7,10,13,16-hexaoxacyclooctadécane à partir d'un mélange contenant aussi un ou plusieurs autres polyéthers macrocycliques contenant des cycles de plus de 7 atomes de carbone, caractérisé en ce qu'on fait réagir le 1,4,7,10,13,16-hexaoxacyclooctadécane avec du nitrométhane en présence d'un solvant pour les polyéthers macrocycliques, de manière à précipiter un complexe de 1,4,7,10,13,16-hexaoxacyclooctadécane et de nitrométhane, on sépare le complexe précipité du mélange contenant le complexe, on dissocie le complexe séparé et on sépare les constituants l'un de l'autre.

2. Procédé selon la revendication 1, caractérisé en ce que le solvant comprend plus de 50% en poids de nitrométhane.

3. Procédé selon la revendication 1, caractérisé en ce que le solvant comprend plus de 50% en poids d'un éther (autre qu'un polyéther macrocyclique ayant plus de 7 atomes de carbone dans le cycle).

4. Procédé selon la revendication 3, caractérisé en ce que l'éther est un oxyde de dialcoyle.

5. Procédé selon la revendication 4, caractérisé en ce que l'oxyde de dialcoyle est de l'oxyde d'éthyle.

6. Procédé selon la revendication 3, caractérisé en ce que l'éther est un éther cyclique.

7. Procédé selon la revendication 6, caractérisé en ce que l'éther cyclique est le 1,4-dioxane.

8. Procédé selon la revendication 6, caractérisé en ce que l'éther cyclique est le tétrahydrofuranne.

9. Procédé selon la revendication 1, caractérisé en ce que le solvant comprend plus de 50% en poids d'un alcanol.

10. Procédé selon la revendication 9, caractérisé en ce que l'alcanol est le méthanol.

11. Procédé selon la revendication 1, caractérisé en ce que le solvant comprend plus de 50% en poids d'un hydrocarbure ou d'un mélange d'hydrocarbures.

12. Procédé selon la revendication 11, caractérisé en ce que l'hydrocarbure est du benzène.

13. Procédé selon la revendication 1, caractérisé en ce que le solvant comprend plus de 50% en poids d'eau.

14. Procédé selon l'une revendications précédentes, caractérisé en ce que la réaction entre le 1,4,7,10,13,16-hexaoxacyclooctadécane et le nitrométhane est conduite à une température comprise entre −25°C et +35°C.

15. Procédé selon la revendication 2, caractérisé en ce que la réaction entre le 1,4,7,10,13,16-hexaoxacyclooctadécane et le nitrométhane est conduite à une température comprise entre 0°C et −25°C.

16. Procédé selon l'une des revendications 3 à 13, caractérisé en ce que la réaction entre le 1,4,7,10,13,16-hexaoxacyclooctadécane et le nitrométhane est conduite à une tempèrature comprise entre 0°C et +35°C.

17. Procédé selon l'une des revendications précédentes, caractérisé en ce que le 1,4,7,10,13,16-hexaoxacyclooctadécane dans le mélange de départ a été obtenu en faisant réagir du tétraéthylèneglycol avec un oxyde de bis(2-halogénoéthyle) en présence d'un hydroxyde de métal alcalin.

## Patentansprüche

1. Verfahren zur Isolierung von 1,4,7,10,13,16-Hexaoxacyclooctadecan aus einem Gemisch, das auch einen oder mehrere andere makrocyclische Polyäther enthält, die Ringe mit mehr als 7 Kohlenstoffatome enthalten, dadurch gekennzeichnet, dass man das 1,4,7,10,13,16-Hexaoxacyclooctadecan mit Nitromethan in Gegenwart eines Lösungsmittels für die makrocyclischen Polyäther mischt, um einen Komplex von 1,4,7,10,13,16-Hexaoxacyclooctadecan und Nitromethan auszufällen, den ausgefällten Komplex von dem den Komplex enthaltenden Gemisch abtrennt, den abgetrennten Komplex aufspaltet und die Komponenten voneinander trennt.

2. Verfahren nach Anspruch 1, dadurch gekennzeichnet, dass das Lösungsmittel mehr als 50 Gew.-% Nitromethan enthält.

3. Verfahren nach Anspruch 1, dadurch gekennzeichnet, dass das Lösungsmittel mehr als 50 Gew.-% eines Äthers (der nicht ein makrocyclischer Polyäther mit mehr als 7 Kohlenstoffatomen im Ring ist) enthält.

4. Verfahren nach Anspruch 3, dadurch gekennzeichnet, dass der Äther ein Dialkyläther ist.

5. Verfahren nach Anspruch 4, dadurch gekennzeichnet, dass der Dialkyläther Diäthyläther ist.

6. Verfahren nach Anspruch 3, dadurch gekennzeichnet, dass der Äther ein cyclischer Äther ist.

7. Verfahren nach Anspruch 6, dadurch gekennzeichnet, dass der cyclische Äther 1,4-Dioxan ist.

8. Verfahren nach Anspruch 6, dadurch gekennzeichnet, dass der cyclische Äther Tetrahydrofuran ist.

9. Verfahren nach Anspruch 1, dadurch gekennzeichnet, dass das Lösungsmittel mehr als 50 Gew.-% eines Alkanols enthält.

10. Verfahren nach Anspruch 9, dadurch gekennzeichnet, dass der Alkanol Methanol ist.

11. Verfahren nach Anspruch 1, dadurch gekennzeichnet, dass das Lösungsmittel mehr als 50 Gew.-% eines Kohlenwasserstoffs oder Gemisches von Kohlenwasserstoffen enthält.

12. Verfahren nach Anspruch 11, dadurch gekennzeichnet, dass der Kohlenwasserstoff Benzol ist.

13. Verfahren nach Anspruch 1, dadurch gekennzeichnet, dass das Lösungsmittel mehr als 50 Gew.-% Wasser enthält.

14. Verfahren nach einem der vorhergehenden Ansprüche, dadurch gekennzeichnet, dass die Reaktion zwischen 1,4,7,10,13,16-Hexaoxacyclooctadecan und Nitromethan bei einer Temperatur im Bereich von −25°C bis +35°C durchgeführt wird.

15. Verfahren nach Anspruch 2, dadurch gekennzeichnet, dass die Reaktion zwischen 1,4,7,10,13,16-Hexaoxacyclooctadecan und Nitromethan bei einer Temperatur im Bereich von 0°C bis −25°C durchgeführt wird.

16. Verfahren nach einem der Ansprüche 3 bis 13, dadurch gekennzeichnet, dass die Reaktion zwischen 1,4,7,10,13,16-Hexaoxacyclooctadecan und Nitromethan bei einer Temperatur im Bereich von 0°C bis +35°C durchgeführt wird.

17. Verfahren nach einem der vorhergehenden Ansprüche, dadurch gekennzeichnet, dass das 1,4,7,10,13,16-Hexaoxacyclooctadecan in dem Ausgangsgemisch erhalten worden ist durch Umsetzung von Tetraäthylenglykol mit einem Bis(2-halogenäthyl)äther in Gegenwart eines Alkalimetallhydroxids.